Europäisches Patentamt

European Patent Office

Office européen des brevets

(11) Publication number: **0 062 032**
**B1**

(12) **EUROPEAN PATENT SPECIFICATION**

(45) Date of publication of patent specification: **21.01.87**

(21) Application number: **80901900.3**

(22) Date of filing: **03.10.80**

(86) International application number:
**PCT/SE80/00241**

(87) International publication number:
**WO 82/01135 15.04.82 Gazette 82/10**

(51) Int. Cl.⁴: **A 61 N 1/42**

(54) **ELECTROMEDICAL TREATMENT APPARATUS.**

(43) Date of publication of application:
· · **13.10.82 Bulletin 82/41**

(45) Publication of the grant of the patent:
**21.01.87 Bulletin 87/04**

(84) Designated Contracting States:
**AT CH DE FR GB LI NL**

(56) References cited:
**GB-A- 871 672**
**US-A-3 915 151**

(73) Proprietor: **ROSENGART, Henning**
**Rullharvsgatan 2E**
**S-431 40 Mölndal (SE)**

(72) Inventor: **ROSENGART, Henning**
**Rullharvsgatan 2E**
**S-431 40 Mölndal (SE)**

(74) Representative: **Roth, Ernst Adolf Michael et al**
**GÖTEBORGS PATENTBYRA AB Box 5005**
**S-402 21 Göteborg (SE)**

Courier Press, Leamington Spa, England.

## Description

The present invention refers to an electromagnetic treatment apparatus for the treatment of biological tissue, preferably for accelerating the healing process in a part of the human body like an arm or a leg, comprising two coaxially arranged, laterally spaced solenoids for generating an alternating magnetic field and two electrodes for generating an alternating electric field which is perpendicular to the said magnetic field, the treatment zone being defined by the region where the magnetic and electric fields cross.

Background of the Invention

A large part of the economic and personnel resources of todays medical service is used for the treatment of patients with long lasting illness periods. As an example we can mention the treatment of fractures such as a fractured thigh bone, treatment of rheumatism and patients with neurologic movement handicaps and long term therapy patients with slow-healing infected wounds etc. For this reason it is not at least of economical value, but also for reducing personal suffering, of great importance to point out and use medical methods and equipment which accelerate and support the healing processes.

It has been known for a long time that magnetic and electric fields affect biological tissue. Injuries and diseases have been treated with varying results. Magnetic and electrical fields have been applied against the diseased or wounded body part and then one has hoped for the best. An example of this kind of treatment apparatus is described in the American patent No. 3,915,151. It is primarily adapted for treatment of broken bones and uses preferably flat coils for generation of a magnetic field whose flow is arranged longitudinally with the treatment object, i.e. parallel to a leg or an arm. In one embodiment an electrostatic field is introduced besides the magnetic field. This is achieved by two diametrically opposite electrodes, to which a voltage with relatively high potential difference is supplied. An electric field is hereby achieved which flows through the treatment object essentially at a right angles to the flow of the magnetic field. By supplying a voltage, whose amplitude fluctuate regularly, the electrically charged particles in the treatment area are brought into an oscillating movement. Due to the flow directions of one magnetic and electric fields, the charged particles oscillate only at right angles to the skeletal structure and thus perpendicular to the main blood flow of the treatment object. The venous blood flow, i.e. the blood flow towards the heart, is thereby not affected by this treatment apparatus.

Another apparatus is described in GB—A—871672. This document describes an apparatus for medical purposes, in which an electric and a magnetic field are produced. These fields are adjustable and variable and can be changed in polarity. The electric and magnetic fields are disposed at right angles to each other in order to produce in the object treated by their simultaneous action an electrodynamic effect which can be brought to an optimum value by the adjustment or variation of the phase relation of the two fields. One application of this apparatus is a short-wave apparatus which produces at the same time an electrical capacitor field and a magnetic coil field in a region which can be selected as desired in the object treated. This apparatus is fed by alternating current which means that the electrolyte will rotate in two different directions owing to the direction of the magnetic field. This means that the electrolyte rotates against the flow direction of the blood in half the cycle and thereby counteract against the circulation of the blood. This also results in that heat is generated in the electrolyte.

Summary of the Invention

The object of the present invention is to accelerate the healing process in biological tissue by bringing the electrically charged particles in the tissue into a helixformed movement which support the metabolism in the tissue and increase the volume of the blood flow. Further objects are to treat rheumatics and patients with circulatory disturbances as well as to prevent development of thrombosis. Patients with neurological disturbances and diseases such as neurologically caused movement disturbances should further be treated with success. The invention should also be easy and safe to use. This is achieved by the fact that at least one magnetic lens and one electric lens are arranged inside and coaxially with the solenoid(s).

Description of the Drawings

An embodiment of the invention will here below be described with reference to the attached drawings.

Fig. 1 shows a sideview in perspective of the electromedical treatment apparatus,

Fig. 2 shows a vertical section through the apparatus,

Fig. 3 shows an electrical circuit diagram of the apparatus,

Fig. 4 shows the design of a lens system,

Fig. 5a, 5b and 5c show some examples of particle paths in electromagnetic fields.

Description of the Embodiment

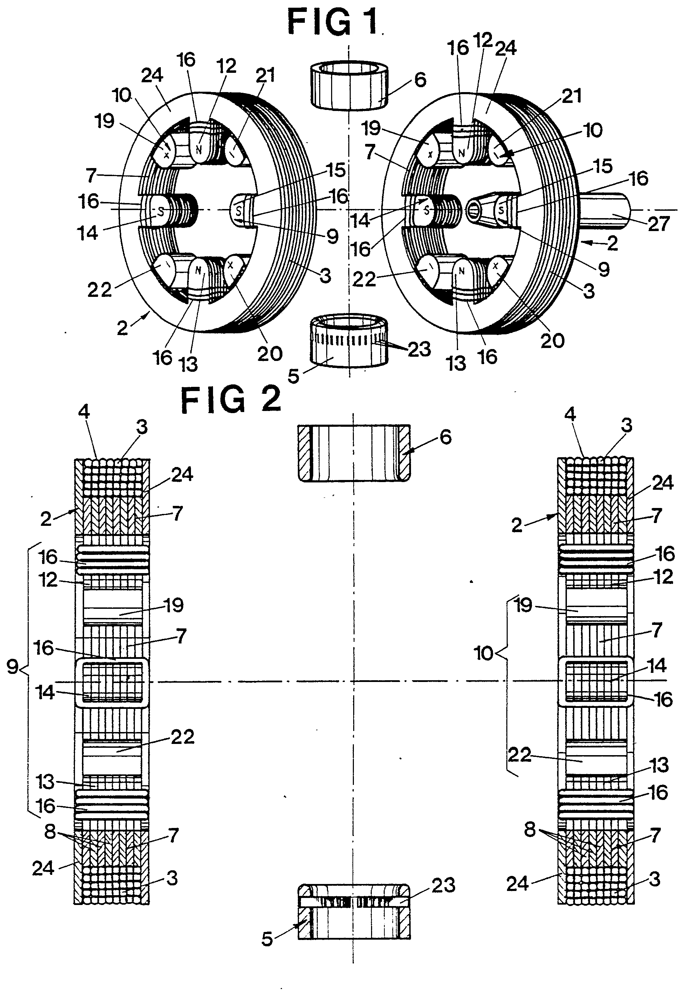

In Fig. 1 there is shown a schematic side view of the electromagnetic treatment apparatus. The parts included are two ringshaped solenoid systems 2 coaxially arranged horizontally and at some distance from one another. Each solenoid system 2 is at its circumference equipped with a solenoid 3. These solenoids 3 are electrically connected in series and form together a magnetic field generator 4. Between the two solenoid systems 2 there is a lower and an upper field electrode 5, 6 arranged in a way, that the central axis in each field electrode 5, 6 coincides with each other. The common central axis of the field electrodes 5, 6 intersects the central axis of the solenoid~system 2 perpendicular and at essentially the same distance from each solenoid system 2. The solenoid systems 2 and the field electrodes 5, 6 are arranged in a way that each of them can manually be adjusted along a horizontal resp. vertical direction with respect to each other. This arrangement results in that the center of the treatment apparatus i.e. the treatment zone between the solenoid system 2 and the field electrodes 5, 6, which is defined by the region where the magnetic and the electric fields cross, can be adjusted to a larger or a smaller size and in this way be adapted to the size of the object to be treated, for example it can be made smaller if an object such as an ankle is to be treated, or larger when a femur is to be treated.

Each of the solenoid systems 2 included in the treatment apparatus consists of a ringshaped core 7, built up of transformer core sheets 8. These sheet metal lamina of for example iron, are in a conventional way isolated from each other and tightly packed in the longitudinal axial direction of the solenoid system 2. In the defined inner space of each ringshaped core 7 is arranged one magnetic and one electric lens 9 resp. 10. The elements 12—15, 19—22 included in each lens 9 resp. 10 can either be attached at the inner surface of the ringshaped core 7 or in another way freely be arranged inside the ringshaped core 7.

At the circumference of the solenoid system 2, on the outer surface of the ringshaped core 7, there is arranged a solenoid 3. The solenoid 3 consists of an isolated copper coiling whose wire diameter is adjusted to the maximum current which is to be fed into the solenoid 3. To keep the coils of the solenoids 3 in place two side pieces 24 of dielectric material are arranged at each side surfaces of the solenoids. In order to maintain a sufficiently strong magnetic field a number of wiring turns may be necessary. Both of the solenoids 3 included in the treatment apparatus are electrically connected in series whereby a homogenous magnetic field is maintained in the treatment zone.

The magnetic lens 9 arranged in the core 7 is of the so called quadrupole type and contains four lens elements 12—15 each of which forms a magnetic pole. The preferably cylindrically shaped lens elements 12—15, which for example can be punched from the same material as the ringshaped core 7, are located symmetrically along the inner surface of the core 7 in such a way that the two lens elements 12, 13 which forms the north poles and the two lens elements 14, 15 which forms the south poles are arranged in pairs diametrically opposite each other. The surface of the lens elements 12—15 turned inwardly towards the center of the magnetic lens 9 are hyperbolically formed. Hereby a more homogenous magnetic field in the center of the magnetic lens 9 is maintained. Around each lens element 12—15 there are arranged lens solenoids 16 in such a manner that the central axis of each solenoid 16 coincides within one and the same point in the center of the solenoid system 2.

The lens solenoids 16 are connected electrically in a way that two diametrically arranged lens elements 14, 15 work as south poles and the other two as north poles. The lens solenoids 16 included in the magnetic lens 9 can be electrically connected in series and connected to a voltage unit 17. The main purpose of the magnetic lens 9 is to converge electrically charged particles which pass through the magnetic lens 9. Its function should, however, be seen in relation to other included parts of the electromagnetic treatment apparatus 1.

Also the electric lens 10 included in the solenoid system 2 is of the so called quadrupole type, i.e. also this has four lens elements here called lens electrodes 19—22. Each lens electrode 19—22 consists of an electrically conducting material, for example copper or the like, and is essentially rod-shaped. The surface facing the center of the solenoid system 2 is preferably hyperbolically shaped in order to maintain the desired field distribution. The lens electrodes 19—22 are symmetrically arranged inside the ringshaped core 7 of the solenoid systems 2, in such a way that the two lens electrodes 19—20 with positive potential and the two lens electrodes 21—22 with negative potential are arranged in pairs diametrically opposite each other. The lens electrodes 19—22 that are electrically insulated from the ring shaped core 7 with insulators 25 are displaced by an angle of 45° relative to the lens elements 12—15 contained in the solenoid system 2. The lens electrodes 21—22 that have a negative potential are connected and wired to the negative terminal of the voltage unit 17 and the lens electrodes 19—20 that have positive potential are connected and wired to the positive terminal of the voltage unit 17. The above described electrical lens 10 diverges the electrically charged particles that pass through its central section.

In the middle of the electromagnetic treatment apparatus, i.e. in its treatment zone, there is arranged an electric field which is directed perpendicular to the magnetic field. This electrical field is generated by an upper and a lower field electrode 6, 5, constituting part of an electrical field generator 26. The upper field electrode 6 consists of a ringshaped pipe of metal whereon the pipe end turned towards the treatment zone is hyperbolically shaped. This upper field electrode 6 is supplied with a positive potential, which results in that the flow of the electrical field is directed downwards.

The lower field electrode 5 consists in the same way as the upper field electrode 6 of metal and is formed as a ringshaped pipe. A grid 23 is arranged at its inner edge. This grid 23 consists of bands or rods of tungsten or molybdenum and are arranged in the upper part of the lower field electrode 5. The upper edges of the grid 23 are formed with a sharp egg-shaped profile and directed towards the upper field electrode 6. Hereby an electric field is obtained, whose flow is focussed toward the inner part of the lower field electrode 5. This results in that a more concentrated field distribution is obtained in the treatment zone.

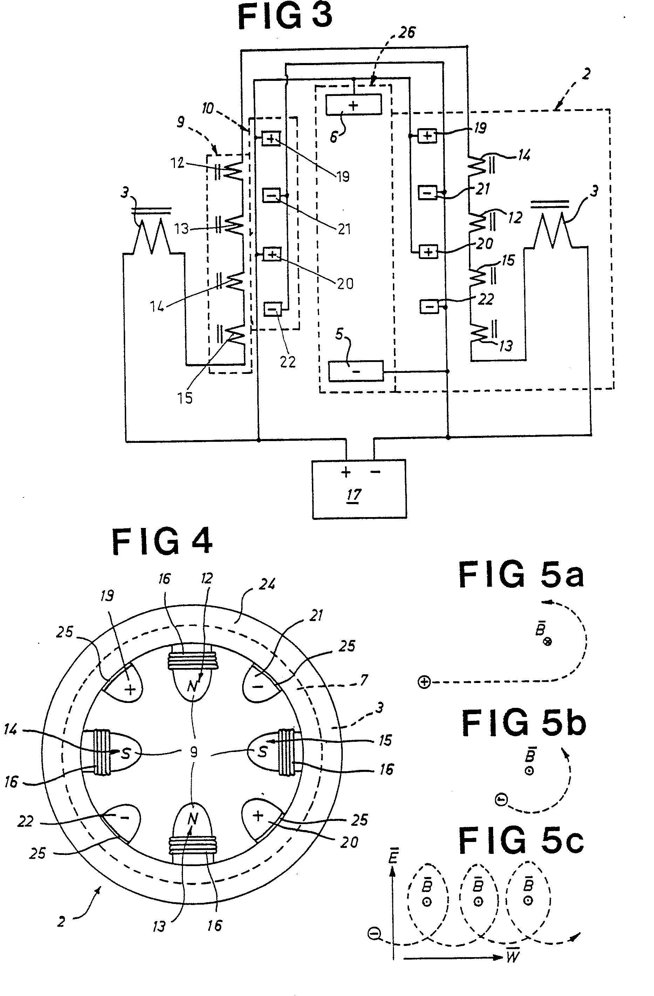

As is shown in Fig. 3 all solenoids 16 arranged at the lens elements 12—15 are connected in series and all lens electrodes 19, 20 with positive potential are connected together and wired to the positive terminal of the voltage unit 17. The lens electrodes 21, 22 are in a related way connected to the negative terminal of the voltage unit 17. One and the same voltage unit 17 supply the entire treatment apparatus 1 with its electrical energy. The voltage consists of a half-wave rectified sinus voltage that never passes zero. The frequency with which the voltage varies has been chosen to 66 Hz, but can naturally vary between for example 20—100 Hz. Also the amplitude of the voltage is freely adjustable. The voltage to the solenoids 3 and 16 can either be positive or negative. It is of course possible to separate the purely electrical system from the electromagnetical system and thereby supply the solenoids 3, 16 on the one hand and the lens electrodes 19—22 and field electrodes 5, 6 on the other hand with voltages whose values differ. It is, however, desirable that the voltage to both systems can vary in accordance to amplitude, frequency and polarity.

In another embodiment for example a pipe or a nozzle 27 for gas or liquid additive can be arranged at one or both ends of the electromagnetic apparatus, i.e. coaxially with the central axis through the solenoid system 2. Through this pipe or nozzle 27, electrically charged particles possibly mixed with one or more medically active substances, can be supplied to the treatment zone via the solenoid systems. In a further embodiment the electromedical treatment apparatus according to the invention comprises one solenoid 3 including a magnetic solenoid system and an electric solenoid system and two electrodes for generating an electric field. The solenoid 3 is thereby located in such a way that its magnetic field crosses the electric field of the electrodes.

Short Theoretical Magnetohydrodynamical Background

The function of the invention is based upon generally accepted physical and chemical laws as well as upon new discoveries within the medical research. The theoretical basis is among others collected from the magnetohydrodynamics and the plasmaphysics disclosed in "Classical Electrodynamics" by Jackson and "Vacuumgaselectronphysics" by Lyman Spitzen, Princeton University. What connects to the medical discoveries reference is made to for example Medicine and Biology, No. 3, 1969.

Fig. 5a—c shows paths of charged particles in different fields according to accepted laws of nature. E and B represent an electric and magnetic field resp. In Fig. 5a is shown the movement path of a positive particle in a magnetic field directed "away from the viewer". In Fig. 5b is shown the movement path of a negative particle in a magnetic field directed "toward the viewer".

Finally is in Fig. 5c shown how a negatively charged particle performs a helically-shaped movement in two, against each other, perpendicular fields, whereby the particle is transported over a distance $\overline{W}$. A magnetic field B directed toward the viewer and an electric field E directed upward (as shown by arrows). It is among other things this movement that is achieved by the present invention.

Function of the Invention

Our blood, as is known, contains a number of different elements and substances. The most common ions, the so called anions and cathions are potassium-, sodium-, chloride-, calcium-, iron-, sulphate and phosphate ions. When an ion is a particle whose resultant charge is either positive or negative, it is influenced by electric and magnetic fields. If the ion passes through one of these fields energy is transferred to it, whereby it moves along paths as shown in Fig. 5a—c. The velocity of the ion depends among other factors on the viscosity of the surrounding substance. Also the ability of the ion to drag with it other particles in the substance affect the mobility. Convection movements in the substance and density gradients can to some extent also affect the movement of the ion.

In a number of injuries and diseases it has been advantageous to use a treatment that causes hyperaemia, i.e. localized oversupply of blood and increased throughflow of blood. This has so far been achieved by means of physiotherapy, whereby mainly different forms of heating apparatuses have been used, such as heating lamps and high frequency techniques. The increased blood flow which has been achieved in this way, assists the transportation of e.g. nutritional elements, proteins and oxygen to the cells and undissolved products from them. This metabolism supports the healing processes. A substantial drawback during treatment with heating lamps is that the infrared radiation can only pass through a few millimeters in the skin and thereby only affects the body surface.

The electromedical treatment apparatus according to the invention is effective also on deeper lying tissue. This is due to that all positive or negative charged particles in an electric or magnetic field are affected and that these particles are spread in all parts of the tissue.

The electromagnetic radiation is intended to affect the bioelectrical potentials on the cellular plane of the organism — cell membranes and mitochondria etc. — but naturally also will affect water distribution in the organism depending on the special structure of the water molecules. Other organic molecules with dipole characteristics will probably also be affected.

When voltage is supplied the treatment apparatus generates a horizontal magnetic field and a vertical electric field the treatment zone being defined by the region where the magnetic and electric fields cross. If charged particles, for example the ions in the blood plasma, are supplied into the treatment zone they are influenced to move in a determined pattern. The path of the ions is influenced by the strength of the magnetic and electric fields. They drag with them uncharged particles in their immediate surroundings. The strength of the fields is by choice adjustable. The magnetic flux density reaches for example $2 \times 10 - 1$ Tesla (2000 Gauss). Both the strength of the magnetic and electric fields vary at the rate of the pulse frequency of the introduced voltage. A pulse frequency of 66 Hz has been shown to coincide with the biological activity of the body but can, when needed, be adjusted between 20—80 Hz.

By locating the relevant bodypart, for example an arm or a leg, in the treatment zone substantially perpendicular to both the magnetic and electric field, the ions perform a helically formed movement in the direction of the length of the blood vessels (see Fig. 5c). When substantially all charged particles have an equal translatory movement component and each charged particle to a greater or lesser extent dregs with it other, uncharged closely lying particles, an increased through flow of blood in the vessels is obtained.

The energy transferred to the ion results in a movement which in short can be described in the following way. A positively charged particle that approaches a magnetic field will curve and rotate counter-clockwise seen in the view of the direction of the flow of the magnetic field. If an electric field is applied perpendicular to the magnetic field and the flow of the electric field is directed upward, the positively charged particle will accelerate onto the right side of the magnetic field. If the particle is negatively charged, the relationship will be the opposite. The final movement path will be helixshaped, i.e. screwformed, with a constant pitch. This is visualized in Fig. 5c.

Due to the fact that the charged particles start to move and thereby bring with them close lying particles, an advantageous increase of the blood throughflow is obtained in the treated tissue. Also an analgesic, i.e. pain reducing, effect can be shown. Pain depends on lack of oxygen in the tissues or of an accumulation of pain substances. An increase of the blood flow gives increased oxygen supply and supports the removal of the pain substances.

Since also the central nervous system works by means of electrically charged particles which among others pass in and out of the nerve cells and in this way transmit information, to for example the muscles, also neurologically caused wounds or diseases can be treated with the electro magnetic treatment apparatus according to the invention. An example of this is that certain types of handicaps, such as neurologically caused movement restrictions, can be treated.

A large part of diseases of older persons stem from the circulatory system and its peripheries. Within this disease area it is difficult to produce adequate medicine because the sideeffects on other organs increase, especially so on older persons. There exists therefore a clear need for an alternative technique that limits its affect on the cellular plane to the diseased organ.

Many persons suffer from so called calcification of the skeleton when they get older. This greatly increases the risks of fractures, where femur breakage is a common injury. The treatment period for this type of injury can be very long and consume a large part of medical service resources. With the electromagnetic treatment apparatus according to the invention a quicker healing is achieved when treating these femur breaks. It has been shown that calcium ions can be forced to wander back into the skeleton whereby a delimed skeleton can be regenerated. In this way bone fractures also can be prevented.

The healing of leg wounds, burns and slow-healing, infected sores can also be accelerated with the treatment apparatus according to the invention. This can for example be carried out by the fact that a medically active substance such as active iodine, or antibiotics in the form of a spray is supplied to the treatment zone by acceleration from one or both of the solenoid systems 2 via for example a nozzle 27. This is also the case with hyperbar oxygentherapy. A bombardment of the wound with the medically active substances can thereby be achieved. Due to this method of treatment the otherwise developing resistant infection causes can be avoided.

**Claims**

1. Electromagnetic treatment apparatus for the treatment of biological tissue, preferably for accelerating the healing process in a part of the human body like an arm or a leg, comprising two coaxially arranged, laterally spaced solenoids (3) for generating an alternating magnetic field and two electrodes (5, 6) for generating an alternating electric field which is perpendicular to the said magnetic field, the treatment zone being defined by the region where the magnetic and electric fields cross, characterized in, that at least one magnetic lens (9) and one electric lens (10) are arranged inside and coaxially with the solenoid(s) (3).

2. Electromagnetic treatment apparatus according to claim 1, characterized in, that at least one nozzle (27) is arranged coaxially with the solenoids (3) to supply the treatment zone with charged particles and/or medically active substance.

3. Electromagnetic treatment apparatus according to claim 1 or 2, characterized in, that the magnetic lens (9) is a quadrupole lens.

4. Electromagnetic treatment apparatus according to any of claims 1—3, characterized in, that electrodes (5, 6) are tube-shaped and have ends facing the treatment zone which are hyperbolically formed.

5. Electromagnetic treatment apparatus according to any one of claims 1—4, characterized in, that the solenoid(s) (3) are electrically connected in series and arranged for power supply from a voltage unit (17).

6. Electromagnetic treatment apparatus according to any of claims 1—4, characterized in, that in one of the electrodes (5, 6) there is arranged a grid (23).

7. Electromagnetic treatment apparatus according to claim 5, characterized in, that the grid (23) is provided by rods or consists of a band of molybdenum or tungsten.

## Patentansprüche

1. Elektromagnetisches Behandlungsgerät für die Behandlung von biologischem Gewebe, insbesondere für die Beschleunigung des Heilungsprozesses in einem menschlichen Körperteil, wie z.B. einem Arm oder einem Bein, bestehend aus zwei koaxial angeordneten, seitlich zueinander im Abstand leigenden Magnetspulen (3) zur Erzeugung eines Magnetwechselfeldes, und zwei Elektroden (5, 6) zur Erzeugung eines zum genannten Magnetfeld senkrecht verlaufenden elektrischen Wechselfeldes, wobei die Behandlungszone durch den Bereich, wo sich das Magnetfeld und das elektrische Feld kreuzen, definiert ist, dadurch gekennzeichnet, daß wenigstens eine magnetische Linse (9) und eine elektrische Linse (10) im Inneren der Magnetspule(n) (3) koaxial zu dieser(n) angeordnet sind.

2. Elektromagnetisches Behandlungsgerät nach Anspruch 1, dadurch gekennzeichnet, daß wenigstens eine Düse (27) koaxial zu den Magnetspulen (3) angeordnet ist, um die Behandlungszone mit geladenen Teilchen und/oder medizinisch aktiver Substanz zu versorgen.

3. Elektromagnetisches Behandlungsgerät nach Anspruch 1 oder 2, dadurch gekennzeichnet, daß die magnetische Linse (9) eine vierpolige Linse ist.

4. Elektromagnetisches Behandlungsgerät nach einem der Ansprüche 1—3, dadurch gekennzeichnet, daß die Elektroden (5, 6) rohrförmig ausgebildet sind und zur Behandlungszone weisende Enden besitzen, die hyperbolisch geformt sind.

5. Elektromagnetisches Behandlungsgerät nach einem der Ansprüche 1—4, dadurch gekennzeichnet, daß die Magnetspulen (len (3) elektrisch in Reihe geschaltet und von einer Spannungsquelle (17) gespeist werden.

6. Elektromagnetisches Behandlungsgerät nach einem der Ansprüche 1—4, dadurch gekennzeichnet, daß in einer der Elektroden (5, 6) ein Gitter (23) angeordnet ist.

7. Elektromagnetisches Behandlungsgerät nach Anspruch 5, dadurch gekennzeichnet, daß das Gitter (23) durch Stäbe gebildet ist oder aus einem Band aus Molybdän oder Wolfram besteht.

## Revendications

1. Appareil de traitement électromagnétique pour le traitement d'un tissu biologique, de préférence pour accélérer le processus de cicatrisation dans une partie du corps humain telle qu'un bras ou une jambe, comportant deux solénoïdes (3) disposés coaxialement et espacés latéralement servant à produire un champ magnétique alternatif et deux électrodes (5, 6) servant à produire un champ électrique alternatif, qui est perpendiculaire audit champ magnétique, la zone de traitement étant définie par la région où le champ magnétique et le champ électrique se croisent, caractérisé en ce qu'au moins une lentille magnétique (9) et une lentille électrique (10) sont disposées à l'intérieur du ou des solénoïdes (3) et coaxialement par rapport à ces derniers.

2. Appareil de traitement électromagnétique selon la revendication 1, caractérisé en ce qu'au moins une buse (27) est disposée coaxialement par rapport aux solénoïdes (3) de manière à alimenter la zone de traitement avec des particules chargées et/ou une substance médicalement active.

3. Appareil de traitement électromagnétique selon la revendication 1 ou 2, caractérisé en ce que la lentille magnétique (9) est une lentille quadripolaire.

4. Appareil de traitement électromagnétique selon l'une quelconque des revendications 1—3, caractérisé en ce que les électrodes (5, 6) possèdent une forme tubulaire et possèdent des extrémités, tournées vers la zone de traitement, qui ont une forme hyperbolique.

5. Appareil de traitement électromagnétique selon l'une quelconque des revendications 1—4, caractérisé en ce que les solénoïdes (3) sont raccordés électriquement en série et sont disposés de manière à réaliser une alimentation en énergie à partir d'une unité (17) délivrant une tension.

6. Appareil de traitement électromagnétique selon l'une quelconque des revendications 1—4, caractérisé en ce qu'une grille (23) est disposée à l'intérieur d'une des électrodes (5, 6).

7. Appareil de traitement électromagnétique selon la revendication 5, caractérisé en ce que la grille (23) est munie de tiges ou est constituée par une bande de molybdène ou de tungstène.

# FIG 1

# FIG 2

0 062 032

# FIG 3

# FIG 4

# FIG 5a

# FIG 5b

# FIG 5c